(19) Europäisches Patentamt
European Patent Office
Office européen des brevets

(11) EP 1 570 048 B1

(12) **EUROPEAN PATENT SPECIFICATION**

(45) Date of publication and mention of the grant of the patent:
**26.09.2007 Bulletin 2007/39**

(21) Application number: **03786108.5**

(22) Date of filing: **15.12.2003**

(51) Int Cl.:
***C12N 1/14*** *(2006.01)* ***A01N 63/04*** *(2006.01)*

(86) International application number:
**PCT/GB2003/005440**

(87) International publication number:
**WO 2004/054359 (01.07.2004 Gazette 2004/27)**

(54) **BIOLOGICAL CONTROL AGENT AND FORMULATIONS**

BIOLOGISCHES SCHÄDLINGSBEKÄMPFUNGSMITTEL UND FORMULIERUNGEN

BIOPESTICIDE ET FORMULATIONS

(84) Designated Contracting States:
**ES GR IT PT TR**

(30) Priority: **13.12.2002 GB 0229127**

(43) Date of publication of application:
**07.09.2005 Bulletin 2005/36**

(73) Proprietor: **A.M.C. Chemical S.L.**
**Poligono Industrial La Isla,**
**Dos Hermanas,**
**41700 Sevilla (ES)**

(72) Inventors:
- **AKDI, Khalid**
  **C/Rio Viejo R 17**
  **E-41700 Sevilla (ES)**
- **PEREZ, Barrera, Francisco**
  **C/Rio Viejo R 17**
  **E-41700 Sevilla (ES)**
- **EXPOSITO, Cubero, Carmelo**
  **C/Rio Viejo R 17**
  **E-41700 Sevilla (ES)**
- **GOMIS, Garcia, Maria, Dolores**
  **C/Rio Viejo R 17**
  **E-41700 Sevilla (ES)**

(74) Representative: **Esteban Perez-Serrano, Maria Isabel**
**UDAPI & Asociados, S.L.,**
**Explanada, 8**
**28040 Madrid (ES)**

(56) References cited:
**EP-A- 0 570 089** **WO-A-92/11856**
**US-A1- 2002 031 495**

- **FARIA MARCOS ET AL: "Biological control of Bemisia tabaci with fungi" CROP PROTECTION, vol. 20, no. 9, November 2001 (2001-11), pages 767-778, XP002288098 ISSN: 0261-2194**
- **LANDA Z ET AL: "A bioassay for determining pathogenicity of entomogenous fungi on whiteflies" BIOLOGICAL CONTROL, vol. 4, no. 4, 1994, pages 341-350, XP002288110 ISSN: 1049-9644**
- **POPRAWSKI T J ET AL: "Effect of host plant on Beauveria bassiana- and Paecilomyces fumosoroseus-induced mortality of Trialeurodes vaporariorum (Homoptera: Aleyrodidae)" ENVIRONMENTAL ENTOMOLOGY, vol. 29, no. 5, October 2000 (2000-10), pages 1048-1053, XP008032789 ISSN: 0046-225X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 570 048 B1

## Description

This invention relates to liquid and solid formulations based on strain *Bb1* of filamentous fungus *Beauveria bassiana,* biological control agent, resistant to commercial insecticides, for the control of pests in plants and crops.

BACKGROUND OF THE INVENTION

Arthropods, especially insects, have an important damaging effect on crops, before as well as after harvesting. Among insects, those belonging to the family *Aleyrodidae* are economically important, especially *Bemisia tabaci* Gennadius and *Trialeurodes vaporariorum* Westwood. The former is a pest of cotton and soybean crops. The latter is usually present in protected horticultural crops (tomato, cucumber, melon, etc.). Both species are commonly known as white fly, referring to the general appearance of adults (winged small insects, covered by white waxy powder). These insects belong to the Order Homoptera, which also includes other important pests, as plant-lice, wood-lice, carrot rust flies, etc. These are biting-sucking insects, living at the expense of plants, feeding on their sap.

*Trialeurodes vaporariorum* Westwood was detected for the first time in Spain in 1943, probably coming from the tropical area in Central America. In 1970 it became one of the most important pests of protected horticultural crops, in a fast expansion process. Since then (in the late eighties), new species, especially *Bemisia tabaci* Gennadius, coming from tropical areas in Asia, have spread out everywhere in Spain. In 1991, both species were present in horticultural crops, being difficult to differentiate in field. Nowadays, whiteflies are still one of the most important horticultural pests.

In order to an effective control of these insects, it is necessary a further knowledge of the biological characteristics of each species.

The significance of whiteflies as a crop pest is mainly due to several biological features that provide them with a high expansion potential. Among these features, can be emphasized: a) *they are highly polyphagic,* feeding on a great number of plant species throughout the world, each insect species with a different host preference; b) *they are highly prolific,* with very fast growth and development rates under advantageous conditions, showing a life cycle between 20 and 30 days, and an egg number per female between 100 and 400; c) *they show an easy dissemination,* due to the mobility of adults spreading to the upper parts of the plants, and between adjacent plants, and in spite of their limited flight capability, they can be carried to long distances by air draughts; d) *they are highly adaptable to variable conditions,* developing in several temperature, humidity, and light conditions, both outdoors and in greenhouses. These factors, as well as the characteristics of the host plant (species, variety, nutritional state, level of infestation, geographical location, etc.) and the activities of natural predators, affect the evolution of the whitefly populations, giving as a result a very variable development.

Crop damage caused by the white fly are usually the result of the suction of leaf tissues and the secretion of a kind of molasses by the larvae or by the adult insects. This can produce the following effects in the crops:

- If the white fly population is very big, the suction of the plant fluids by the adult can affect the plant physiological processes, and prevent growth. With intense sunlight, leaves can wither and fall. Leaf damages affect the development of the fruit and can originate a harvest decreasing.
- Plant elaborated sap is too rich in carbohydrates for the requirements of the flies. For that reason they have to eliminate the excess as a liquid secretion (molasses) which is deposited on the crop. This molasses also serves as food to a saprophytic fungus known as "fumagina" *(Cladosporium spherosporum)* or "negrilla" which is easily developed. Both the molasses and the saprophytic fungi decrease photosynthetic activity and plant transpiration, originate bad appearance fruits, with a less commercial value, and involve extra manipulation costs. On the other hand, they serve as refuge to other pests and diseases.
- White fly acts as a transmission vector for important virosis in crops, mainly horticulturals. Larvae or adults, infected by feeding on a damaged plant, hold the virus, which reproduces inside. The insects can infect a great number of plants during their life. Among the more of 1000 known species of white fly, only three have been confirmed as transmission vectors of viruses (*T. vaporariorum, T. abutilon* and *B. tabaci*). *T. vaporariorum* propagates several viruses to horticultural and fruit crops, such as the lettuce infectious yellow virus. *B. tabaci* is particularly effective in propagating virosis, being the most important in Spain the tomato yellow leaf curl virus (TYLCV). A major risk of disease occurs in summer and autumn, when the highest population sizes are reached.

Since its appearance as an economically important pest, the control of the white fly has been one of the main problems in agriculture. Nevertheless, an evolution in the control strategy has been observed throughout the years, due to variations in the white fly species. At the present time, the control of the white fly involves high costs of the crops. For that reason, the choice of a suitable strategy and application conditions is an important factor in pest control.

Some cultural and preventive methods designed to avoid the focuses of the phytophage and the crop infections are advised, like: elimination of weeds, use of meshes and elimination of vegetal remains of previous harvests. In any case, a great care must be taken for not eliminating the natural predators using these methods.

Chemical control of this pest is very problematic because of the fast acquisition of resistance to all type of pesticides. Commonly used products are methomyl (only for outdoors treatments), imidacloprid and some development regulators like buprofezin. Other products are: some pyrethroids, naled, oil, endosulfan etc.

Insect control with chemical pesticides has not been always satisfactory. Thus, there have been cases in which insect elaborates from his cuticle a wax which prevents the penetration of the pesticides, allowing the fast development of resistant populations and decreasing the control effectiveness of the pesticides.

Biological control is being used as an alternative to chemical control. There are many references about natural enemies of the white fly, like parasites and predators, as well as pathogens. Thus, aphelinid hymenopter *Encarsia formosa* has been used in greenhouses for a long time.

For an effective biological control it is necessary to consider the following points:

- Search and potentiation of native natural enemies. Species of parasites, like *E. mundus,* have shown high parasitism levels against *B. tabaci* in tomato crops.
- Take into account the influence of weeds and crop-adjacent areas for the mobility of the pest and its natural enemies.
- Compatibility between chemical products and natural enemies.

Concerning pest management, biological control usually refers to the action that parasites, predators or pathogens have on the pest population, giving rise to its decrease below the damaging levels, maintaining it in natural balance within the ecosystem.

*Parasites* are those organisms that live in or on the body of another organism during part of their life.

*Predators* are those free-living animals that feed on other animals. Predators can attack their prey both in the immature as in the adult state; as example birds, reptiles, mammals, and arthropods.

*Pathogens* are those microorganisms useful for the pest control. Thus, as major agents of control, are emphasized:

- bacteria: like *Bacillus thuringensis* against caterpillars, beetles, mosquitoes, etc
- fungi: like *Metarhizium* (against cockroaches); *Beauveria bassiana* (against white fly, Colorado potato beetle, corn rootworms, etc.) and *Verticillium lecanii.*
- viruses: like nucleopolyhedrosis viruses (against European corn borer)
- protozoa: like *Nosema locustae* (against lizards, grasshoppers)
- nematodes: like *Steimesema* and *Heterorhabditis* (against soil weevils, stem-boring caterpillars).

There are a great number of fungi reported, that can live as parasites and kill different types of arthropods, breaking their cuticle, and penetrating through their hard and resistant exoskeleton.

Among all organisms considered as biological control agents against pests, entomopathogenic fungi are those that offer a major chance.

There are more than 500 species with known pathogenic capability, but only a small number of these are used in biological control. This is mainly because the way of penetration, through the cuticle, can be affected by environmental conditions, being humidity essential for the first steps of penetration.

Fungal species more commonly used in biological control are *Beauveria bassiana, Meterhizium anisopliae* and *Verticillium lecanii.* These species have a worldwide distribution and a wide host range. Other notable species, associated to a certain kind of host, are *Hirsutella thompsonii,* specific of ticks (arachnids), and aquatic fungi of genera like *Coelomyces* spp. and *Culicinomyces* spp., specific to mosquitoes.

Parasitic fungi of insects or other arthropods can act during all different host development stages. Therefore, these fungi can be applied in every interesting stage.

Pest natural enemies (parasites, predators or pathogens) should be applied at the beginning of the culture cycle, when plants are still young, pest incidence is low and damage is not observable. Since biological control agents act slower than pesticides, they cannot be used as a recovery treatment.

Deuteromycetes grow well in solid media, producing high amount of conidia, which are the infective units. These conidia germinate, and the hyphae penetrate through the cuticle, invading epidermis and hypodermis and spreading out in host tissues, resulting in the death of the insect. Then, the fungus lives as a saprophyte in the body of the insect, producing new conidia that disseminate and germinate again, completing the life cycle.

In order to an effective utilization of fungi for biological control, it is necessary a high amount of inoculum to obtain a short-term and short-span effect. This effect usually endures only one season, it being necessary to repeat the treatment periodically.

Entomopathogenic fungi cause the host death rapidly by producing a great amount of spores or conidia that can give rise to an important epizooty and destroy the pest. Since these epizooties are affected by environmental factors, it is difficult to predict the success of inoculations in natural environments.

*Beauveria bassiana* is a pathogen of insects that are commonly found in nature, mainly in some plants as well as in soil. Among its hosts, whiteflies (*Trialeurodes vaporariorum* and *Bemisia tabaci*), homoptera belonging to family *Aleyrodidae,* can be emphasized. Other host species are coleoptera, hemiptera, heteroptera, lepidoptera and diptera, in temperate as well as in tropical areas.

The biological cycle of *B. bassiana* includes two phases, a pathogenic phase and a saprophytic phase. Pathogenesis occurs when the fungus comes into contact with live tissues of the host, being necessary a microclimate humidity of 85 % or above. *B. bassiana* is a facultative parasite. Infective process, carried out by conidia (fungal reproductive structures), comprises three steps. First stage is the germination of conidia, followed by a penetration and development of hyphae inside the insect (3 or 4 days). This process can take place through the cuticle (by enzymatic secretions: lipases, chitinases and proteases) or orally. Conidial germ tube invades by producing apresoria that penetrate the epicuticle, giving rise to fruiting bodies. Fungal pathogenicity on insects depends on a complex relationship between fungal capability to penetrate the cuticle, and the resistance of the insect immunological system to prevent fungal development.

Fungal development inside the insect can be influenced by the effectiveness of the hematocytes to encapsulate and melanize the pathogen. Hematocytes form aggregates in the penetration site giving rise to "nodules" around the conidia. Inside the insect body, germination only occurs when conidia are outside the hematocyte aggregates.

In the second stage, insect tissues are invaded by fungal mycelium, giving as a result the insect death (2 to 3 days). In this process, the fungus produces many toxic secondary metabolites. Among them, beauvericine, beauverolides, bassianolides, isarolides, enniatines and oosporein can be emphasized. Disease symptoms in the insect are loss of sensitivity and movement coordination, and finally paralysis.

Third stage is the sporulation and the beginning of a new cycle. Fungal mycelium, which can be firstly observed in the joints and the soft parts of the insect, spreads out in all the body, covering it, and causing therefore the death of the insect.

In most entomopathogenic fungi, initial infection steps include a direct penetration through the insect cuticle. Therefore, ultrastructural and histochemical evidences suggest that the process involves an enzymatic degradation; indeed, it has been shown that these fungi can produce enzymes like lipases, proteases and chitinases.

It has been shown that serinprotease Pr1 is the majority enzyme in the degradation of the cuticle, which protein content is more than 70%. The effect of Pr1 has been demonstrated by a significant cuticle degradation and by its high concentration in the penetration sites.

It has been shown that Pr1 has a different substrate specificity in each fungal species. These differences in the affinity for the Pr1 binding sites in each species are one of the main factors in the recognition of the insects to infect.

Isolation and characterization of *Pr1* gene and its ORF has been achieved by molecular studies using RACE (Rapid Amplification of cDNA Ends) approaches. Sequences of Pr1 genes from different species have been compared and their aminoacid homology has been established. These results will be very useful for fungal identification.

Integrated Pest Management (IPM) consists in the utilization of biological control approaches together with other chemical and physical techniques, in order to reduce the environmental impact caused by the latter.

A successful application of IPM strategies involves the maintenance of healthy crops, for which routine examinations are necessary, in order to detect early infections and take the appropriate corrective measures. Among these measures, the following are remarked:

- Monitoring of crops.
- Use of entomopathogenic fungi, biological micopesticides, can be characterized by:
    - ➢ Host specificity
    - ➢ Low production costs
    - ➢ Activity in a wide range of environmental conditions
    - ➢ Non human pathogenicity.

The first step in biological control application is the search, isolation and selection of the antagonists by morphological, physiological, biochemical and molecular studies, in order to their adequate characterization before field application.

A second important step is an exhaustive study for the selection and development of appropriate culture media for fungal biomass production.

The massive production of biological control agents has been discussed by diverse authors throughout several decades. This industrial process involves the production of conidia or other starters implied in the antagonism. They should be produced in cheap, available media, with the suitable nutrient balance and as less time as possible.

Substrates used for the industrial production of entomopathogenic fungi are mainly cereal grains, either broken or not, supplemented with specific nutrients, or inert clays as vermiculite impregnated with culture media. Once selected the appropriate substrates, the optimal fermentation conditions must be established (temperature, light, humidity, ventilation, shaking, etc.).

Another requirement is the obtaining of sufficient biomass, with the adequate starters for its effectiveness.

The following stop would be the formulation stage. A good formulation for biological control must have the following characteristics:

- Base of preparation
- Stability ,
- Apppoptiats survival rates
- Abundant viable starters
- Low cost

The formulation must have an important role in the protection of the antagonists during their storage and application, as well as facilitate their spreading and growth, and assure their persistence in field. In addition it must allow an easy application of the product without loss, of effectiveness. Therefore, to formulate the micoinsecticide it is necessary to avoid those processes or practices that can inhibit or damage it.

In the casa of *B. bassiana,* the commercial development of an effective formulation must consider the necessity for preserving its viability and virulence during the production process.

It is known from the State of the art the "Biological Control of *Bemisia tabaci* with fungi" as the one disclosed in the document Faria Marcos et al.: "Biological control of Bemisia tabaci with fungi". This document reviews the latest innovations in the biological control of *Bemisia tabaci* using fungi. In addition to isolation and selection techniques, the authors describe some commercial products proven to be effective for the biological control of tobacco white fly both in the greenhouse and in the open field crops. The strain of the invention can withstand extreme conditions of pH and temperature, such as pH equal to 10 and temperatures of 75°C during five minutes it was effective at controlling adult insects in integrated control strategies using adulticides compatible with the fungus and it has been selected on the basis of superproduction of extracellular enzymes such as proteases, glucanases and chitinases.

SUMMARY OF THE INVENTION

The present invention provides filamentous fungus *Beauveria bassiana* strain *Bb*1 deposited under CECT 20480.

In other aspects, the invention provides formulations comprising *B. bassiana, Bb1* (CECT 20480) and a carrier.

In further aspects, the invention provides the use of *B. bassiana, Bb1* (CECT 20480) or the use of the formulations as mentioned above, as biological control agents, for example of pests in plants and crops, in particular against white fly (*Bemisia tabaci, Trialeurodes vaporariorum* and *Aleyrodes proletella*).

In further aspects, the invention provides methods of using, or the use of, *B. bassiana, Bb1* (CECT 20480) or formulations as mentioned above, in combination with, or synergistically with, compatible chemical insecticides such as Orytis®, Dicarzol®, Lannate® and Confidor®, or in combination with, or synergistically with, Previeur® chemical fungicide at half the maximum concentration recommended by the manufacturer.

DETAILED DESCRIPTION OF THE INVENTION

*Isolation of the Beauveria bassiana strain*

*Beauveria bassiana Bb1* strain, object of this invention, was isolated and selected from the sample of an infected host. Monosporic and pure cultures were obtained in the laboratory.

The isolated strain was stored in a solution of 25% glycerol at -80 °C. The strain is easily recovered in potato-dextrose-agar (PDA), com-meal-agar (CMA) and malt-extract-agar (MEA).

This strain has been deposited in the Colección Española de Cultivos Tipo (CECT). Table 1 shows the internal code of the laboratory, the assigned accession number and the date of deposit.

TABLE 1

| Internal code | CECT number | Date of deposit |
|---|---|---|
| *Bb1* | 20480 | 15 April 2002 |

*Morphological characterization*

From the previously obtained monosporic cultures, the different morphological characteristics in laboratory culture media, like PDA, CMA and MEA, were studied. The cultures were incubated at 25°C in darkness during 14 days. The types of mycelium and conidial development were studied, and slides in lactophenol cotton blue were made for microscopic examination.

*Physiological and Metabolic Characterization*

*Carbon source assimilation*

Capacity of assimilation of different carbon sources by *Bb1* strain was studied. Tested carbon sources were: glucose (LBGL), lactic acid (LBAL), citric acid (LBCA), ammonium oxalate (LBAO) and ammonium tartrate (LBAT).

Lynch B liquid medium ($NH_4H_2PO_4$ 1 g/l, KCl 0.2 g/l, $MgSO_4.7H_2O$ 0.2 g/l, $CuSO_4.5H_2O$ 5 mg/l and $ZnSO_4.7H_2O$ 10 mg/l) containing 0.05 g/l of bromocresol purple, and 1% (w/v) of the tested carbon source, was used. The medium was adjusted to pH 6. 10 ml of medium were dispensed in slant tubes. Media with the different carbon sources were autoclaved. The tubes were inoculated with 0.5 cm (2%) water-agar plugs of the strain. All experiments were made in triplicate. Results were observed after incubating the tubes in darkness at 25°C, during 14 days.

Table 2 shows the results obtained for carbon source assimilation. As observed, *Bb1* strain can not assimilate ammonium oxalate neither ammonium tartrate as carbon sources.

TABLE 2

| | | *Bb1* |
|---|---|---|
| LBGL | Slant growth | + |
| | Radial growth | +++ |
| | Sporulation | +++ |
| | Medium alkalinization | - |
| LBLA | Slant growth | + |
| | Radial growth | +++ |
| | Sporulation | ++ |
| | Medium alkalinization | - |
| LBCA | Slant growth | ++ |
| | Radial growth | ++ |
| | Sporulation | + |
| | Medium alkalinization | + |
| LBAO | Slant growth | - |
| | Radial growth | - |
| | Sporulation | - |
| | Medium alkalinization | - |
| LBAT | Slant growth | - |
| | Radial growth | - |
| | Sporulation | - |
| | Medium alkalinization | - |

+++: strongly positive, ++: very positive, +: positive, (+): weakly positive, ((+)): very weakly, -: negative, n: non tested

*Nitrogen source assimilation*

Capacity of assimilation of different nitrogen sources by *Bb1* strain was studied. Tested nitrogen sources were: ammonium oxalate (LAAO), sodium nitrite (LANI) and glycine (LAGY).

Lynch A liquid medium ($KH_2PO_4$ 1 g/l, KCl 0.5 g/l, $MgSO_4.7H_2O$ 0.2 g/l, $CaCl_2.2H_2O$ 0.1 g/l and sucrose 10 g/l) containing 0.05 g/l of bromocresol purple, and 0.2% (w/v) of the tested nitrogen source, was used. The medium was adjusted to pH 6. 10 ml of medium were dispensed in slant tubes. Media with the different nitrogen sources were autoclaved. The tubes were inoculated with 0.5 cm (2%) water-agar plugs of the strain. All experiments were made in triplicate. Results were observed after incubating the tubes in darkness at 25°C, during 14 days.

TABLE 3

| | | *Bb1* |
|---|---|---|
| LAAO | Slant growth | + |
| | Radial growth | +++ |
| | Sporulation | +++ |
| | Medium alkalinization | - |
| LANI | Slant growth | (+) |
| | Radial growth | (+) |
| | Sporulation | - |
| | Medium alkalinization | - |
| LAGY | Slant growth | ++ |
| | Radial growth | +++ |
| | Sporulation | +++ |
| | Medium alkalinization | - |

+++: strongly positive, ++: very positive, +: positive, (+): weakly positive, ((+)): very weakly, -: negative, n: non tested

Table 3 shows the results obtained for nitrogen source assimilation. As observed, *Bb1* strain can assimilate the different tested nitrogen sources.

*Growth on different microbiological culture media*

Different culture characteristics of *Bb1* strain on different culture media were studied. Tested culture media were: water agar (AA), malt extract agar (MEA), corn meal agar (CMA), CZ $NH_4$ agar, glycerol nitrate agar (G25N) and sucrose nitrite agar (ANS).

Plates were prepared with the different tested media, and inoculated with 0.5 cm (2%) water agar plugs of the strain. Plates were incubated in darkness at 25°C during 14 days. All experiments were made in triplicate.

Table 4 shows the results obtained after 14 days of incubation of *Bb1* strain in the different media.

## TABLE 4

| | | *Bb1* |
|---|---|---|
| AA | Colony diameter (cm) | 5.3 |
| | Sporulation | (+) |
| | Aerial mycelium | - |
| M | Colony diameter (cm) | 5.87 |
| | Sporulation | + |
| | Aerial mycelium | + |
| CMA | Colony diameter (cm) | 6.6 |
| | Sporulation | (+) |
| | Aerial mycelium | - |
| $CZNH_4$ | Colony diameter (cm) | 3.3 |
| | Sporulation | - |
| | Aerial mycelium | v |
| G25N | Colony diameter (cm) | 1.8 |
| | Sporulation | - |
| | Aerial mycelium | - |
| ANS | Colony diameter (cm) | 5.4 |
| | Sporulation | - |
| | Aerial mycelium | + |

+++: strongly positive, ++: very positive, +: positive, (+): weakly positive, ((+)): very weakly, -: negative

*Effect of chemical growth inhibitors*

The effect of chemical reagents cupric sulfate, zinc sulfate and crystal violet on *Bb1* strain growth was studied. Tested concentrations were the following:

- Crystal violet: 0.2 g/l and 0.04 g/l
- Cupric sulfate: 0.5 g/l
- Zinc sulfate: 3 g/l

Solutions of these chemical reagents were sterilized by filtration (Millipore HA of 0.45 μm) and were added to flasks containing 250 ml of autoclaved Lynch B agar medium. Media with the different reagent concentrations were dispensed in Petri dishes and inoculated with 0.5 cm (2%) water-agar plugs of the strain. All experiments were made in triplicate. Inoculated plates were observed after 24 hours, 48 hours and 7 days of incubation in darkness at 25°C.

➢ *Cupric sulfate:*

*Bb1* strain growth was completely inhibited when this reagent was present.

➢ *Crystal violet:*

*Bb1* strain grew in Lynch B medium with all different tested concentrations of crystal violet, during 7 days. Colonies are smooth, hyaline, without sporulation.

➢ *Zinc sulfate:*

*Bb1* strain grew in Lynch B medium with zinc sulfate during 7 days. Colonies are smooth, hyaline, without sporulation.

*Gelatine hydrolysis*

Gelatine hydrolysis was studied, as well as *Bb1* strain sporulation, in a gelatine rich medium, after 10 days of incubation.

Gelatine rich medium contains: sucrose 10 g/l, gelatine 120 g, $CuSO_4.5H_2O$ 5 mg/l, $ZnSO_4.7\ H_2O$ 10 mg/l, stock solution A 50 ml/l, stock solution C 50 ml. Stock solution A formulation is: $NaNO_3$ 40 g/l, KCL 10g/l, $MgSO_4.7\ H_2O$ 10 g/l, $FeSO_4.7\ H_2O$ 0.2 g/l. Stock solution C formulation is: $K_2HPO_4$ 20 g/l.

Medium was dispensed in flasks, autoclaved and inoculated with 0.5 cm (2%) water agar plugs of the strain. Flasks were incubated in darkness at 25°C during 10 days and maintained at a 4°C for one hour.

Table 5 shows the results obtained after 10 days of incubation of *Bb1* strain in gelatine rich medium.

TABLE 5

| | *Bb1* |
|---|---|
| Growth | ++ |
| Gelatine hydrolysis | (+) |

+++: strongly positive, ++: very positive, +: positive, (+): weakly positive, ((+)): very weakly, -: negative

*Esculin hydrolysis*

Esculin hydrolysis by *Bb1* strain was studied. Petri dishes containing esculin agar were inoculated with 0.5 cm (2%) water agar plugs of the strain. Inoculated cultures were incubated in darkness at 25°C during 10 days. The experiment was made in triplicate.

Hydrolysis reaction is considered as positive when colony reverse turns to black. Hydrolysis produces glucose and esculetine (which interacts with ferric ion producing a black precipitate that darkens the medium).
Table 6 shows the results obtained after 10 days of incubation of *Bb1* strain.

TABLE 6

| | *Bb1* |
|---|---|
| Growth | ++ |
| Esculin hydrolysis | + |

+++: strongly positive, ++: very positive, +: positive, (+): weakly positive, ((+)): very weakly, -: negative

*Bb1* strain shows a positive esculin hydrolysis reaction..

*Lipase hydrolysis*

Capacity of lipase hydrolysis, and therefore lipase metabolization, by *Bb1* strain was studied. TWH basal medium (fungal peptone 10 g/l, NaCl 5g/l, $CaCl_2.2H_2O$ 0.1 g) containing bromocresol purple 0.025 g/l, agar 2% (w/v) and 100 ml of a 10% (w/v) Tween 80 solution. The medium was adjusted to pH 6 and was autoclaved. Then, it was dispensed in Petri dishes and inoculated with 0.5 cm (2%) water agar plugs of the strain. Inoculated cultures were incubated in darkness at 25°C during 14 days. The experiment was made in triplicate.

Hydrolysis reaction is considered as positive when medium turns to blue and a white crystalline ring appears under the colonies

Table 7 shows the results obtained after 14 days of incubation.

TABLE 7

| | *Bb1* |
|---|---|
| Growth | ++ |
| Lipase hydrolysis | ((+)) |

+++: strongly positive, ++: very positive, +: positive, (+): weakly positive, ((+)): very weakly, -: negative

*Urease test*

Growth capacity in a medium containing urea was tested. Lynch B medium supplemented with 2 % (w/v) urea, 1% (w/v) glucose, 2% (w/v) agar and 0.05 g/l of bromocresol purple was prepared. The medium was dispensed in Petri dishes after sterilization to establish a comparison with the medium without urea. The plates were inoculated with 0.5 cm (2%) water agar plugs of the strain. Plates were incubated in darkness at 25°C for 14 days. The experiments were made in triplicate.

TABLE 8

| | | Bb1 |
|---|---|---|
| Urea control | Colony diameter (cm) | 1.72 |
| | Sporulation | +++ |
| | Acidification | +++ |
| Urea Test | Colony diameter (cm) | 5.2 |
| | Sporulation | +++ |
| | Alkalinity | (+) |

+++: strongly positive, ++: very positive, +: positive, (+): weakly positive, ((+)): very weakly, -: negative

*Beauveria bassiana tolerance to extreme pH values*

Tolerance to extreme pH values of *Beauveria bassiana* was studied in acid media (pH 2) as well as in basic media (pH 10). The following culture media were made. CZ-pH2 (50 ml/l of CZ A solution, 1 g/l of $K_2HPO_4$, 30 g/l of sucrose, 10.5 g/l of citric acid, 5 mg/l of $CuSO_4.5H_2O$ and 10 mg/l of $ZnSO_4.7\ H_2O$) and CZ-pH10 (50 ml/l of CZ A solution, 3.75 g/l of glycine, 0.2 g/l of $K_2HPO_4$, 30 g/l of sucrose, 5 mg/l of $CuSO_4.5H_2O$ and 10 mg/l of $ZnSO_4.7\ H_2O$) both media were complemented with 0.05 g/l of bromocresol purple. The CZ A solution composition is: 40 g/l of $NaNO_3$, 10 g/l of KCl, 10 g/l of $MgSPO_4.7\ H_2O$ and 0.2 g/l of $FeSO_4\ .\ 7H_2O$.

TABLE 9

| | | Bb1 |
|---|---|---|
| PH 2 | Slant growth | - |
| | Radial growth | - |

| | | |
|---|---|---|
| | Sporulation | - |
| PH 10 | Slant growth | (+) |
| | Radial growth | +++ |
| | Sporulation | +++ |

+++: strongly positive, ++: very positive, +: positive, (+): weakly positive, ((+)): very weakly, -: negative

*Conidial resistance of Beauveria bassiana Bb1 strain to heat.*

Conidia of *Beauveria bassiana Bb1* were heated at 75°C for 5 minutes. Cultures were grown in MEA or PDA for 8 days. A sterile 0.2 % (v/v) Tween 80 solution is added to plates once the fungus has sporulated. Plates were shaken lightly helping conidial dispersion. At the same time slant tubes were prepared adding 1 ml of 0.2 % (v/v) Tween 80 solution and autoclaved. Slant tubes were kept 30 minutes in a water bath at 75°C for temperature stabilization. Then, each tube was inoculated with 100 μl of the conidial suspension and kept for another 5 minutes in the 75°C water bath. Conidial survival was determined by streaking MEA or PDA plates. Plates were incubated in darkness at 25°C for 7 days. The experiment was made in centuplicate.

TABLE 10

| | | *Bb1* |
|---|---|---|
| 75 C | Viability | ++ |

++: fungal growth in 50% of evaluated plates.

*Biochemical characterization*

*Enzymatic activities using API ZYM galleries*

API ZYM system (Biomérieux S.A., Marcy-l'Etoile, France) is a semiquantitative kit for enzymatic activity detection, which allows a rapid simultaneous detection up to 19 enzymatic activities, from small amount of sample. API-ZYM strips are composed of 20 microtubes containing a buffer with an enzymatic substrate.

The following enzymatic activities were studied: Alkaline phosphatase, esterase, esterase lipase, lipase, leucine arylamidase, valine arylamidase, cystine arylamidase, trypsin, α-chymotrypsin, acid phosphatase, naphtol-AS-BI-phosphohydrolase, α-galactosidase, β-galactosidase, β-glucuronidase, α-glucosidase, β-glucosidase, N-acetyl-β-glucosaminidase, α-manosidase y α-fucosidase.

Inoculated samples in the API ZYM strips were *Beauveria bassiana Bb1* strain cultures supernatants grown in darkness, at 25°C for 9 days in GYM medium. The reading of the strips valued from 0 to 2. Table 11 shows the results of the enzymatic activity tests. 0 corresponds to a negative reaction, 2 to a reaction of maximum intensity and 1 corresponds to an intermediate reaction.

TABLE 11

| | *Bb1* |
|---|---|
| Alkaline phosphatase | 0 |
| Esterase | 1 |
| Esterase lipase | 1 |
| Lipase | 0 |
| Leucine arylamidase | 0 |
| Valine arylamidase | 0 |
| Cystine arylamidase | 0 |
| Trypsin | 0 |
| α-chymotrypsin | 1 |
| Acid phosphatase | 2 |
| Naftol-A-S-BI-phosphohydrolase | 2 |
| α-galactosidase | 1 |
| β-galactosidase | 2 |
| β-glucuronidase | 0 |
| α-glucosidase | 1 |
| β-glucosidase | 1 |
| N-acetyl-β-glucosaminidase | 1 |
| α-manosidase | 0 |
| α-fucosidase | 1 |

*Extracellular enzymatic activities*

The following extracellular enzymatic activities were determined: β-1,3-glucanases, β-1,6-glucanases, β-1,4-endoglucanases (CMCases) and exochitinases for the Bb1 *Beauveria bassiana* strains grown in GYM, GYM-CMC and LB-CMC media. (GYM: glucose 10g/l, $NH_4H_2PO_4$ 1g/l, KCl 0.2g/l, $MgSO_4.7H_2O$ 0.2g/l, $CuSO_4.5H_2O$ 5 mg/l y $ZnSO_4$. 7 $H_2O$ 10 mg/l, yeast extract 5g; GYM-CMC is a modification of the GYM medium, changing glucose by carboximethylcellulose (CMC, medium viscosity Sigma) at 1%); LB-CMC ($NH_4H_2PO_4$ 1g/l, KCl 0.2g/l, $MgSO_4$. $7H_2O$ 0.2g/l, $CuSO_4$. $5H_2O$ 5 mg/l and $ZnSO_4$.7 $H_2O$ 10 mg/l, carboximethylcellulose at 1%), in darkness at 25°C during 9 days without shaking.

β-*1,3-glucanase activity*

It is determined at 37°C, in a reaction containing 0.2 ml of a Laminarin solution (β-1,3-glucane proceeding from the red seaweed *Laminaria hyperborea,* Sigma) at 0.5 % (in acetic/ acetate buffer 100 mM at pH 5.2) and 0.1 ml of the enzymatic solution properly diluted. The reaction mix was incubated during one hour and reaction was stopped by heating at 100°C for 7 minutes.

Sugar concentration was determined on 0.5 ml of mixture by Somogyi's (1952) and Nelson's (1957) method, using glucose at 0 to 1 mg/ml as standard.

An enzymatic activity unit of β-1,3-glucanase was defined as the amount of enzyme able to produce one micromol of glucose equivalents per minute in test conditions.

Sugar determination was made as following: 0.15 ml of Somogyi I: Somogyi II (4:1) solution was added to 0.15 ml of our sample. The mixture was boiled for 10 minutes and then ice cooled, 0.15 ml of a Nelson solution was added and strongly shaken in order to help $CO_2$ removal. Finally, mixture was diluted in 1.5 ml of distilled water and absorbance at 540 nm was read in a microplate reading luminometer (Elx 800, Bio-Tek Instruments, Inc).

The following solutions were used:

*Somogyi I solution:* 15 g of sodium potassium tartrate and 30 g of anhydrous $Na_2CO_3$ was diluted in 300 ml of distilled water. 20 g $NaHCO_3$ were added afterwards. In a different tube 180 g of anhydrous $Na_2SO_4$ was diluted in 500 ml of distilled water heating without boiling in order to remove air formed. Once cooled, the latter solution was added to the former and the volume up to 1 litre was filled with distilled water. Final mixture was stored at room temperature.

*Somogyi II solution:* 2 g of $CuSO_4$. $5H_2O$ and 18g of anhydrous $Na_2SO_4$ were dissolved in 100 ml of distilled water. Reagent was stored at room temperature.

*Nelson solution:* 12.5 g of tetrahydrated ammonium molybdate was dissolved into 225 ml of bidistilled water, afterwards 10.5 ml of concentrated $H_2SO_4$ was added while shaking. 1.5 g sodium arsenate was diluted in 12.5 ml of bidistilled water and was mixed with the former mixture. This mixture was preserved from light at room temperature after storing it at least 48 hours at 37°C.

Results of β-1,3-glucanase enzymatic activity (IU/ml) corresponding to *Bb1* strain of *Beauveria bassiana* are shown in table 12 for three different culture media (GYM, GYM-CMC and LB-CMC).

β-*1,6-glucanase activity*

The activity was determined at 37°C. The contents of the mixture of the chemical reaction were: 0.2 ml of a Pustulan (β-1,6-glucan from *Umbillicaria papulosa,* Calbiochem, USA) 0.5% solution (in a potassium acetate 50 mM buffer, pH 5.5) and 0.1 ml of an enzymatic solution properly diluted. The mixture was incubated for one hour and the reaction was stopped heating at 100°C for 7 minutes. Finally, sugar concentration was determined in 0.15 ml of the mixture following the Somogyi-Nelson method (above described), using glucose at 0 to 1 mg/ml as standard.

An enzymatic activity unit of β-1,6-glucanase was defined as the amount of enzyme able to produce one micromol of glucose equivalents per minute in test conditions.

Results of β-1,6-glucanase enzymatic activity (IU/ml) corresponding to *Bb1* strain of *Beauveria bassiana* are shown in table 12 for three different culture media (GYM, GYM-CMC and LB-CMC).

*Cellulasic activity (*β*,4-endoglucanase)*

The activity was determined at 37°C. The contents of the mixture of the chemical reaction are: 0.2 ml of a carboxymethylcellulose (0.5% solution in a potassium acetate 50 mM buffer, pH 5.5) and 0.1 ml of an enzymatic solution properly diluted. The mixture was incubated for one hour and the reaction was stopped heating at 100°C for 7 minutes. Finally, sugar concentration was determined in 0.15 ml of the mixture following the Somogyi-Nelson method (above described), using glucose at 0 to 1 mg/ml as standard.

An enzymatic activity unit of β-1,4-endoglucanase is defined as the amount of enzyme able to produce one micromol of glucose equivalents per minute in test conditions.

Results of β-1,4-endoglucanase enzymatic activity (IU/ml) corresponding to *Bb1* strain of *Beauveria bassiana* are shown in table 12 for three different culture media (GYM, GYM-CMC and LB-CMC).

*Exochitinase activity (*β*-N-acetylglucosaminidase)*

This enzyme hydrolyzes the terminal monomeric units of the chitin. Its activity was calculated measuring the nitrophenol liberation from the p-nitrophenil-β-D-N-acetylglucosaminida (Sigma).

The reaction mixture containing 0.05 ml of a p-nitrophenil-β-D-N-acetylglucosaminidase solution (0.3 mg/ml potassium phosphate $KHPO_4$ 50 mM buffer, pH 6.7) and 0.03 ml of the enzymatic solution properly diluted, was prepared. The plate was incubated for 15 minutes at 50°C and the reaction was stopped adding 0.05 ml of $Na_2CO_3$ 0.4 M. The evaluation was made measuring the absorbance using 96-well plates luminometer at 410 nm. The enzymatic activity was calculated by: A = Absorbance x dilution factor

Table 13 shows the results of the exochitinase enzymatic activity corresponding to the *Beauveria bassiana Bb1* strain in GYM culture medium. In GYM-CMC and LB-CMC culture media, induction of the exochitinase was nearly null.

TABLE 12

*Enzymatic Activities (IU/ml)*

| | β-*1,3-glucanase* | β-*1,6 glucanase* | β-*1,4-endoglucanase* |
|---|---|---|---|
| GYM | 2.224 | 0.251 | 0.016 |
| *GYM-CMC* | 0.167 | 0.086 | 0.300 |
| *LB-CMC* | 0.419 | 0.217 | 0.188 |

*TABLE 13*

| | *Exochitinase activity AU* |
|---|---|
| *GYM* | 4.967 |

AU arbitrary unit AU = Absorbance 410 nm x dilution factor

*Molecular characterization*

*Beauveria bassiana Bb1* strain object of this patent, is a natural isolate, which genome has not been modified. However, the high morphological similarities among the species belonging to the genus *Beauveria* make very difficult a correct identification and discrimination between biological control agents. The morphological methods (*Stasz et al.,* 1988) have resulted without enough resolution to: a) differentiate and characterize industrial strains for patent or commercialization. b) control the genetic stability of the biological control agents during the industrial processes. c) monitor the persistence of antagonistic strains, once they are introduced in the soil and after several successive treatments. d) the fight against fraud and possible litigations of industrial rights.

A great advance was the development of techniques based on biochemical analysis, also known by biochemical markers, which has allowed surpassing some of the mentioned problems. However, these techniques have the disadvantage of not being sufficiently reproducible and resolutive in strain differentiation.

At the moment, the use of nucleic acids (DNA) analysis has been increased, in order to solve some of these difficulties, looking for differences in the fungal genome, biological control agents, which could be reproducible molecular markers, allowing the identification of the different strains

Proper techniques are necessary in order to find differences within the organism DNA. The most powerful tools for this purpose are those based in the polymerase chain reaction (*PCR*) described by Mullis and Fallona (1987).

One of the potential methodologies are those based on the amplification of DNA fragments or genes with a specific highly conserved sequence, using PCR. In filamentous fungi, especially in species belonging to genus *Beauveria,* the rRNA genes are highly conserved and very similar through different related species. Nevertheless, the ITS region (Internal Transcribed Spacer), allow us to establish important and precise differences at infraspecific level. These regions could be amplified by ITS 1 and ITS4 primers (Kuhls *et al.,* 1996).

A necessary condition for molecular characterization of *Beauveria bassiana Bb1* strain is working with genetically pure populations, considering the great phenotypic variability exhibited by the fungal isolates cultured in laboratory.

*Genomic DNA extraction*

Genomic DNA extraction from the *Beauveria bassiana Bb1* strain was made following the method proposed by Raeder and Broda (1985) with some modifications.

*Solid media:* For maintaining fungal cultures and starters, plates with PDA medium (Potato Dextrose Agar, Sigma-USA) were prepared. 0.5 cm agar plugs from other *Bb1* strain cultures were transferred at the centre of the plate using an sterile cloning cylinder. Plates were incubated in darkness at 25°C, until total colonization.

*Liquid media:* Flasks with sterile PDB (Potato Dextrose Broth, Sigma-USA) were inoculated with a conidial suspension ($10^6$ conidia/ml) coming from cultures grown in solid medium (PDA). Inoculated flasks were incubated with shaking, at 25°C and 250 rpm.

All material was previously autoclaved. Fungal mycelium was collected and filtered through a filter paper funnel. Then, was washed several times with distilled sterile water until complete removal of agar remains. The biomass obtained was dried using sterile filter paper and was distributed in criotubes for freezing and conservation.

50 mg of freeze dryed mycelium (freeze dryer, Telstar Cryodos) was triturated in liquid nitrogen, on an ice bath. Ground mycelium was collected in a microtube, and was homogenized in 0.5 ml of lysis buffer (SDS 0.5 %, Tris-HCl 200 mM, NaCl 250 mM, EDTA 25 mM; at pH 8.5). In order to remove proteins from the liquid phase, 350 μl of phenol were added (Phenolic solution was prepared according to Sambrook *et al.,* 1989. Crystallized phenol (Merk) saturated with 1M Tris-HCl pH 8 buffer was used, in presence of 8-hydroxyquinoleine, and stored at 4°C in a sterile topaz glass bottle) and 150 μl of isoamylic chloroform (chloroform/isoamylic alcohol 24/1 (v/v)), mixing carefully. Microtubes were centrifuged at 13000 rpm and 4°C, for 1 hour. The supernatant was transferred to a clean microtube, 500 μg of RNAse were added (10 mg/ml of pancreatic ribonuclease (Sigma) dissolved into a 10 mM Tris-HCl and 15 mM NaCl buffer pH 7.5, and heated up to 100°C during 15 minutes to reactivate it and was aliquoted and conserved at -20°C). Then, it was incubated during 30 minutes at 37°C. An equivalent volume of isoamylic chloroform was added and mixed. After centrifuging for 10 minutes at 13000 rpm at room temperature, the supernatant was transferred to a clean microtube. DNA was precipitated, adding 0.1 volumes of sodium acetate 3M pH 5 and 2.5 volumes of 96% (v/v) ethanol at 20°C. The pellet obtained by centrifugation at 13000 rpm during 10 minutes at 4°C, was washed with cold 70% (v/v) ethanol, and dried to the air and resuspended into 100 ml of TE buffer (10:1) (10 mM Tris-HCl; 1 mM EDTA, pH 8). Genomic DNA samples were stored at -20°C.

*Genomic DNA quantification and purity*

Genomic DNA quantification, as well as its purity was measured by spectrophotometric techniques, following the protocol described in Sambrook *et al.* (1989). 1/100 dilutions of the genomic DNA samples were made with distilled water. Later, absorbances at wavelengths λ=260 nm and λ=280 nm were registered respectively. DNA purity was confirmed by dividing both absorbance values. Result must be higher or equal to 1.8. The amount of genomic DNA was determined considering that, when absorbance λ=260 nm equals 1, the quantity of double stranded genomic DNA is 50 μg/ml (Sambrook *et al.,* 1989).

Genomic DNA extraction yields following the protocol described by Raeder and Broda (1985) were very high.

*Amplification of the ITS region using PCR*

The following primers were used:

*ITS1: 5'-TCG GTA GGT GAA CCT GCG-3'*
*ITS4: 5'-TCC TCC GCT TAT TGA TAT GC- 3'*

Primers are oligonucleotides, which enable the binding of the DNA polymerase enzyme over the DNA template. They are automatically synthesized by Roche Biochemicals, and are provided freeze dryed. They were resuspended in our laboratory into MiliQ (Millipore) water at 50 μM final concentration and then aliquoted in several tubes and stored at -20°C.

*Amplification conditions*

Amplification of the ITS region, was performed using ITS1 and ITS4 primers with a Ptc-100 of MJ Research thermal cycler which incorporates a heated lid to avoid evaporations, using DNA polymerase enzyme (Echo Taq Biochemical) and following the protocol described in White *et al.* (1990) modified. The reagents for the PCR reaction are shown in Table 14:

TABLE 14

| Reagents | Volume | Final concentration |
|---|---|---|
| MiliQ sterile water | - | Up to 50 μL |
| Buffer 10X | 5 μL | 1 X |
| MgC12 (50 mM) 10X solution | 1.5 μL | 1 X |
| Nucleotide mixture 12.5mM | 1 μL | 200 μM each one |
| ITS1 and ITS4 (50 μM) primers | 0.4 μL | 0.4 μM each one |
| Eco Taq polymerase (5U/μL) | 1 μL | 1 U/ 50 μL final volume |
| Genomic DNA template | - | 1 μg |

All the reagents were added following the order shown in Table 14 and mixed in 200 μL PCR-microtubes. All was done in complete sterile conditions in order to avoid any possible contamination, as well as ice to prevent DNA denaturation, annealing of the primers and premature enzymatic activity.

Reaction mixture was centrifuged during 5 seconds and incubated in the thermal cycler for 5 minutes at 95°C (initial denaturation step), later 40 cycles were programmed following the steps below:

- Denaturation of the double stranded DNA 1 min at 95°C
- Binding of the primers to the DNA template 2 min at 55°C
- Extension step 3 min at 72°C

After 40 cycles one final extension cycle was carried, for 10 minutes at 72°C to complete elongation of all strands.

*Genomic DNA and PCR products separation using electrophoresis on agarose gel*

Genomic DNA as well as PCR amplification products were separated and purified by electrophoresis on 1 % agarose gel in TAE (Tris-acetate 40 mM, EDTA 2 mM, pH 8.3) buffer. The electrophoresis was performed under a constant electric current (40 volts) during 4 hours. Samples were loaded on gel, adding ¼ of the sample volume in gel loading buffer (0.25% bromophenol blue, 0.25% Xylene cyanol and 25% Ficoll 400).

At the end of the electrophoresis, gels were stained with a 0.5 μg/ml ethidium bromide solution, photographed and analysed under a photographic-densitometric system (BIOPROFIL) with an ultraviolet transilluminator and a CCD-camera.

*ITS region sequencing*

ITS region PCR fragments from *Beauveria bassiana Bb1* strain were sequenced by using an automatic DNA sequencer (Perkin Elmer/Applied Biosystem). 300 ng of the PCR product and 5 pmols of the following primers were used:

*ITS1: 5' - TCG GTA GGT GAA CCT GCG G-3'*
*ITS2: 5'-GCT GCG TTC TTC ATC GAT GC-3'*
*ITS3 : 5'- GCA TCG ATG AAG AAC GCA GC-3'*
*ITS4: 5'-TCC TCC GCT TAT TGA TAT GC-3'*

Nucleotide sequences obtained were aligned using Meg Align computer application from DNASTAR software package (DNASTAR, London). Database searches were done by service provided by EMBL/Gen Bank (European Bioinformatics Institute, EBI) to compare these sequences with other sequences belonging to the genus *Beauveria.*

Sequenciation using ITS1, ITS2, ITS3 and ITS4 primers, of the ITS region, shows a high homology to other filamentous fungi, as well as with other *Beauveria bassiana* strains from Gen Bank. The existing differences were found in ITS1 and ITS2 spacers. The sequence obtained from the *Beauveria bassiana Bb1* strain is here shown.

*Beauveria bassiana Bb1* CECT 20480 (ITS region).

```
  1  CCCTTTCCCC CGGGCTTTTT TAAATTTGGG AATTAATGGG CCTTTAAAGT
 51  TTTCCAGCGG GGTTAGTCCT AACCTGAATT TCGGAGGTCT AACGTTCAAG
101  GAAGTTGGGT GTTTTACGGC GTGGCCGCGT CGGGGTTCCG GTGCGAGCTG
151  TATTACTACG CAGAGGTCGC CGCGGACGGG CCGCCACTCC ATTTCAGGGC
201  CGGCGGTGTG CTGCCGGTCC CCAACGCCGA CCTCCCCCAG GGGAGGTCGA
251  GGGTTGAAAT GACGCTCGAA CAGGCATGCC CGCCAGAATG CTGGCGGGCG
301  CAATGTGCGT TCAAAGATTC GATGATTCAC TGGATTCTGC AATTCACATT
351  ACTTATCGCA TTTCGCTGCG TTCTTCATCG ATGCCAGAGC CAAGAGATCC
401  GTTGTTGAAA GTTTTGATTC ATTTGTTTTG CCTTGCGGCG TATTCAGAAG
451  ATGCTGGAAT ACAAGAGTTT GAGGTCCCCG GCGGGCCGCT GGTCCAGTCC
501  GCGTCCGGGC TGGGGCGAGT CCGCCGAAGC AACGATAGGT AGGTTCACAG
551  AAGGGTTAGG GAGTTGAAAA CTCGGTAATG ATCCCTCCGC AGGTTCACCT
601  ACGGA
```

*In vitro compatibility of Beauveria bassiana strain with different commercial chemical fungicides and insecticides.*

Biological control agents survival to chemical treatments, conferring them a complementary effect and a reinforcement of their properties. A detailed treatment plan throughout the disease development period can only be done once the pesticide tolerance has been established.

Main benefits of the integration of biological control agents in a pest integrated management program would be:

- A diminution in chemical fungicide and insecticide use. Consequence of this will be a smaller accumulation of pesticide residues in the environment.
- The less the pathogens are exposed to chemical fungicides the less resistances will be acquired by them and therefore, virulence and damage severity in crops will be lessened.

*Insecticide compatibility*

*In vitro* compatibility of *Beauveria bassiana Bb1* strain with the most used chemical insecticides in agriculture as pest control, was tested. This test shows the possibility of using both types of products at the same time, in case a simultaneous treatment with chemical and biological insecticides would be required.

Studies were carried out in order to find if these products would produce an inhibitory growth effect in *Beauveria bassiana Bb1* strain. Different PDA plates were prepared adding the different chemical insecticides. The insecticides concentration was 1 μl/ml (typical dose used in field). At the same time, control plates were prepared, without chemical insecticide. Plates were inoculated using 0.5 cm PDA plugs of *Beauveria* cultures. Plates were incubated at 25°C in darkness for 21 days. The experiment was made in triplicate. The radial inhibition percentage was measured at 3, 7, 14 and 21 days.

The results are given using the following formula:

$$\text{Radial inhibition percentage} = [(C-T)*100]/C$$

Where C is the average of the three control strains and T is the average of the three strains treated with each product (see table 15).

TABLE 15

| | | *Bb1* |
|---|---|---|
| 3 days | Confidor® | 0 |
| | Lannate® | 0 |
| | Dicarzol® | 0 |
| | Orytis® | 0 |
| 7 days | Confidor® | 0 |
| | Lannate® | 0 |
| | Dicarzol® | 0 |
| | Orytis® | 0 |
| 14 days | Confidor® | 0 |
| | Lannate® | 0 |
| | Dicarzol® | 0 |
| | Orytis® | 0 |
| 21 days | Confidor® | 0 |
| | Lannate® | 0 |
| | Dicarzol® | 0 |
| | Orytis® | 0 |

There was no growth inhibition observed in *Bb1* strain colonies while insecticides were present The only possible conclusion is that there is a total compatibility between the tested insecticides and *Beauveria bassiana Bb1* strain.

*Fungicide Compatibility*

The test was made using two different fungicide concentrations: The maximum and minimum doses advised by the manufacturer. Culture medium used was Lynch B (LB) with 1% glucose as carbon source. Inoculums used were PDA-agar plugs of *Bb1* strain from two days old plates grown at 24°C, Two kind of plates were prepared, one containing the fungicide with the different concentrations used in the test and one without it (treated plates and control plates). Plates were incubated at 25°C in darkness for 21 days. The experiment was made in triplicate.

The inhibition percentage was measured at 3, 7, 14 and 21 days. The results are given using the formula:

$$\text{Inhibition percentage} = [(C-T)*100]/C$$

Where C is the average of the three control strains and T is the average of the three strains treated with each product (see tables 16A and 16 B).

TABLE 16A

| | | Bb1 (inhibition %) |
|---|---|---|
| 7th day | Captan® 0.3 % | 100 |
| | Ridomil® 4 g/l | 60.4 |
| | Folicur® Combi 0.3 % | 100 |
| | Tisar® 0.3 % | 100 |
| | Baycor® Z 0.3 % | 100 |
| | Benomyl 100g/l | 100 |
| | Metaxyl 0.3 % | 100 |
| | Tachigaren 2 cc/l | 100 |
| | Previcur® 0.5 % | 72.64 |
| | Scala® 0.2 % | 60.38 |
| | Terraclor® 0.25 cc/l | 72.20 |
| 14th day | Captan® 0.3 % | 100 |
| | Ridomil® 4 g/l | 78.40 |
| | Folicur® Combi 0.3 % | 100 |
| | Tisar® 0.3 % | 100 |
| | Baycor® Z 0.3 % | 100 |
| | Benomyl 100g/l | 100 |
| | Metaxyl 0.3 % | 100 |
| | Tachigaren 2 cc/l | 100 |
| | Previcur® 0.5 % | 53.80 |
| | Scala® 0.2 % | 61.80 |
| | Terraclor® 0.25 cc/l | 72.20 |
| 21st day | Captan® 0.3 % | 100 |
| | Ridomil® 4 g/l | 74.70 |
| | Folicur® Combi 0.3 % | 100 |
| | Tisar® 0.3 % | 100 |
| | Baycor® Z 0.3 % | 100 |
| | Benomyl 100g/l | 100 |
| | Metaxyl 0.3 % | 100 |
| | Tachigaren 2 cc/l | 100 |
| | Previcur® 0.5 % | 67.50 |
| | Scala® 0.2 % | 65.10 |
| | Terraclor® 0.25 cc/l | 80.78 |

TABLE 16B

| | | *Bb1* |
|---|---|---|
| 7th day | Captan 0.15 % | 100 |
| | Ridomil 2 g/l | 56.60 |
| | Folicur Combi 0.15 % | 100 |
| | Tisar 0.15 % | 100 |
| | Baycor Z 0.15 % | 100 |
| | Benomyl 50g/l | 100 |
| | Metaxyl 0.15 % | 100 |
| | Tachigaren 1 cc/l | 100 |
| | Previcur 0.2 % | 0 |
| | Scala 0.1% | 56.60 |
| | Terraclor 0.125 cc/l | 76.90 |
| 14th day | Captan 0.15 % | 100 |
| | Ridomil 2 g/l | 64.20 |
| | Folicur Combi 0.15 % | 100 |
| | Tisar 0.15 % | 100 |
| | Baycor Z 0.15 % | 100 |
| | Benomyl 50g/l | 100 |
| | Metaxyl 0.15 % | 100 |
| | Tachigaren 1 cc/l | 100 |
| | Previcur 0.2 % | 0 |
| | Scala 0.1% | 62.74 |
| | Terraclor 0.125 cc/l | 76.90 |
| 21st day | Captan 0.15 % | 100 |
| | Ridomil 2 g/l | 66.30 |
| | Folicur Combi 0.15 % | 100 |
| | Tisar 0.15 % | 100 |
| | Baycor Z 0.15 % | 100 |
| | Benomyl 50g/l | 100 |
| | Metaxyl 0.15 % | 100 |

| | |
|---|---|
| Tachigaren® 1 cc/l | 100 |
| Previcur® 0.2 % | 0 |
| Scala® 0.1% | 63.30 |
| Terraclor® 0.125 cc/l | 76.90 |

According the results obtained there is no compatibility between the *Bb1* strain and the fungicide maximum recommended concentration. However, when testing with the minimum recommended one (approximately 50% of the maximum concentration) it was only in Previcur that total compatibility was observed during the experiment.

The present invention is further illustrated by the following non-limiting examples which make reference to the accompanying figures.

FIGURES OF THE INVENTION

Figures 1 to 3 show glucanase activity results reported in Table 12.

EXAMPLES OF THE INVENTION

According to above disclosure, liquid and solid formulations are will now be described for their application on leaves and/or soils. The formulation will contain a suspension of vegetative and reproductive structures (conidia) from *Beauveria bassiana Bb1* strain, a biological control agent characterized in this invention.

EXAMPLE 1

Present invention gives a liquid formulation which quantitative composition is as follows:

- Vegetal oils 94-96 %
- Inert components 6-4 %
- *Beauveria bassiana* CECT 20480 c.s.p. $2 \times 10^3$-$2 \times 10^9$ conidia/ml.

It is a mixture of different non-ionic surfactants and emulsionants with vegetal oils. As was exposed in the invention, the formulation has components that are used like nutrients by *Beauveria bassiana Bb1* strain, and maintains a sufficient humidity for the germination of the conidia.

The characteristics of oils present in the formulation let them work as an adhesive and/or antievaporant, which will allow the persistence of the conidia in insects, plants and leave surfaces, retaining the applied product and preventing its volatilization.

Due to the characteristics previously described, the formulation has demonstrated to be very effective in pest control, which attack the plants that have been hardly controlled until now by liquid formulations.

Vegetal oils used in this formulation are a mixture of emulsionants coming from extracts of plants like aloe vera, soybean, jojoba, etc., or esters of fatty acids and glycerol. The inert fraction of the formulation is composed by tensioactive agents like sorbilene, polyethylene glycol, etc.

Liquid formulation viability will be of 85 % after six months of storage in refrigerated conditions, nevertheless viability after the same time of storage at room temperature will only be of 50 %.

Measurement of formulation viability was made by counting colonies. In order to accomplish this, serial dilutions of the formulation were prepared, afterwards 100μl of the solution was grown in PDA (potato, dextrose, agar) supplemented with 0.1 % of Triton 100x. Four plates of each dilution were prepared and were incubated at 25°C for a period of 48 to 72 hours, after this time a colony counting was made. Result was expressed in c.f.u. per ml of product (c.f.u. = colony forming unit).

The formulation was tested in a greenhouse in order to prevent infection of pepper crops by the white fly (*B. tabaci).* Tests were carried out in two different varieties: red California, roxy cultivar (PV1) and yellow lamuyo, maribel and triana cultivars (PV2).

The biological formulation is applied at a concentration ranging from 0.5 to 1 litre per hectare. Simultaneously the formula is applied with two insecticides, one of them is an adulticide and the other one is a chitin inhibitor (IGR). Both insecticides were applied at the manufacturer recommended concentrations.

Formulas were applied using a spraying mesh, with an average concentration of 600 litres per hectare.

Work was carried out in the following way:

- Initial count of white fly levels in crops, counting adults and grubs were carried out. In order to accomplish this 10 % of the greenhouse plants were sampled, looking for flies at three different levels, at the top and the bottom of the plant and in an intermediate level.
- After the initial counting, the tested formulas were applied.
- After five days counting were repeated.
- Counting were repeated four times during the test with 10 days intervals.

Results are expressed in percentage of the initial counting.

TABLE 17

| | F1 | | F1+IGR | | F1+Adulticide | |
|---|---|---|---|---|---|---|
| | Ap | Lp | Ap | Lp | Ap | Lp |
| P V1 | 30 | 22.8 | 65.2 | 20 | 78.3 | 19 |
| P V2 | 28.2 | 19.1 | 35 | 17.4 | 65 | 25.9 |

F1: Example 1 formulation
P V1: Red California pepper, roxy cultivar
P V2: Yellow lamuyo pepper, maribel and triana cultivars
Ap: White fly adult parasitized
Lp: White fly grub parasitized

White fly pest control was observed to be more successful in tests made with simultaneous application of the F1 formula and the IGR product and were even more successful in the test made with the mixture of the formula and the adulticide product. This was due to shock treatments with both adulticide and IGR products do not affect the *Beauveria bassiana* entomopathogenic fungi growth, but they considerably diminish the white fly population.

EXAMPLE 2

Present invention gives a solid formulation composed by a carrier and a combination of biological compounds which quantitative composition is as follows:

*Carrier*

- Protein 10-50 %
- Fats 10-30 %
- Cellulose 1-10 %
- Lactose 10-60 %
- A vitamin 10000-30000 UI/Kg
- D-3 vitamin 1000-10000 UI/Kg
- E vitamin 10-60 mg/Kg
- Cupric sulphate ($Cu\ SO_4.5H_2O$) 5-20 mg/Kg

*Biological compounds*

- *Beauveria bassiana* CECT 20480 c.s.p. $2\times10^3$-$2\times10^9$ conidia/ml.

Carrier characteristics help the formulation action by increasing longevity and viability of the *Beauveria bassiana Bb1* strain conidia because it enhances water absorption and facilitates a conidial suspension in a liquid medium at the application moment.

Adhesive properties of the carrier carbohydrate composition enhances (as in example 1) the conidial adhesion to insect and leave surfaces.

The carrier itself may be used as a nutritive source by *Beauveria bassiana.* At the same time it makes a favourable environment in which the microorganism can establish and survive. The most important characteristic of the carrier is the protection that gives against solar rays, increasing genetic stability of the formulation isolate and help it to retain its efficiency as well as increase viability once applied at field.

Formulation has demonstrated great stability during storage due to the combined effect of all this characteristics.

Solid formulation viability was 90 % after six months of storage at 4-9°C, nevertheless viability after the same time of storage at room temperature was 60 %

Viability was determined following the method described in example 1.

The formulation was tested in a greenhouse in order to prevent infection of tomato crops by the white fly (*B. tabaci*). Tests were carried out in two different varieties: "suelto pintón", brillante cultivar (TV1) and "tomate en ramillete", pitenza cultivar (TV2).

The biological formulation is applied at a concentration ranging from 125 to 250 grams per hectare. Simultaneously the formula was applied with two insecticides, one of them is an adulticide and the other one is a chitin inhibitor (IGR). Both insecticides were applied at the manufacturer recommended concentrations.

Formulas were applied using a spraying mesh, with an average concentration of 600 litres per hectare.

Work was carried out in the following way:

- Initial count of white fly levels in crops, counting adults and grubs were carried out. In order to accomplish this 10 % of the greenhouse plants were sampled, looking for flies at three different levels, at the top and the bottom of the plant and in an intermediate level.
- After the initial counting, the tested formulas were applied.
- After five days counting were repeated.
- Counting were repeated four times during the test with 10 days intervals.

Results are expressed in percentage of the initial counting.

TABLE 18

| | F2 | | F2+IGR | | F2+Adulticide | |
|---|---|---|---|---|---|---|
| | Ap | Lp | Ap | Lp | Ap | Lp |
| TV1 | 39.6 | 20.1 | 50 | ns | 100 | ns |
| T V2 | 45 | 27.3 | 50 | ns | 40 | ns |

F2: Example 2 formulation
T V1: "suelto pintón" tomato, brillante cultivar
T V2: "tomate en ramillete" tomato, pitenza cultivar
Ap: White fly adult parasitized
Lp: White fly grub parasitized

White fly pest control was observed to be more successful in tests made with simultaneous application of the F1 formula and the IGR product and were even more successful in the test made with the mixture of the formula and the adulticide product. This was due to shock treatments with both adulticide and IGR products do not affect the *Beauveria bassiana* entomopathogenic fungi growth, but they considerably diminish the white fly population.

REFERENCES

Kuhls, K., Lieckfeldt, E., Samuels, G.J., Kovacs, W., Meyer, W., Petrini, O., Gams, W., Börner, T. and Kubicek, C.P. (1996). Molecular evidence that the asexual industrial fungus Trichoderma reesei is a clonal derivate of the ascomycete Hypocrea jecorina. Proceedings of the National Academy of Sciences, U.S.A.

Mullis, K.B. and Fallona, F.A. (1987). Specific synthesis of DNA in vitro via polymerase-catalyzed chain reaction. Methods in Enzymology 155: 335-350.

Nelson, N.J. (1957). Colorimetric analysis of sugars. In Methods in enzymology. Vol. III. (eds. S.P. Colowick and N. Kaplan). New York: Academic Press pp 85-86.

Raeder , U. and Broda, P. (1985). Rapid preparation of DNA from filamentous fungi. Letters Applied Microbiology 1: 17-20.

Sambrook, J., Fritsche, E. and Maniatis, T. (1989). Molecular cloning: A laboratory manual. Cold Spring Harbor: Cold Spring Harbor Laboratory.

Somogyi, M. (1952). Notes on sugar determination. Journal Biological Chemistry 195:19-29.

White, T.J., Bruns, T., Lee, S. and Taylor, J. (1990). Amplification and direct sequencing of fungal ribosomal RNA gens for phylogenetics. In PCR Protocols-A Guide to Methods and Applications. (ed. M.A. Innis et al.). San Diego: Academic Press pp. 315-322.

## Claims

1. Filamentous fungus *Beauveria bassiana* strain *Bb1* deposited under CECT 20480, adaptable to a basic extreme pH value of 10.

2. A filamentous fungus according to claim 1 adaptable to an extreme temperature of 75°C during five minutes.

3. The use of *B. bassiana* strain *Bb1* deposited under CECT 20480 as a biological control agent.

4. A formulation comprising filamentous fungus B. bassiana strain *Bb1* deposited under CECT 20480, and a carrier.

**5.** A liquid formulation which quantitative composition is as follows:

- Vegetal oils 94-96 wt%
- Inert components 6-4 wt%
- *Beauveria bassiana* CECT 20480 c.s.p. $2\times10^3$-$2\times10^9$ conidia/ml

**6.** A solid formulation composed by a carrier and a combination of biological compounds which quantitative composition is as follows:
*Carrier*

- Protein 10-50 wt%
- Fats 10-30 wt%
- Cellulose 1-10 wt%
- Lactose 10-60 wt%
- A vitamin 10000-30000 UI/Kg
- D-3 vitamin 1000-10000 UI/Kg
- E vitamin 10-60 mg/Kg
- Cupric sulphate ($CuSO_4$ $5H_2O$) 5-20 mg/Kg

*Biological compounds*
- *Beauveria bassiana* CECT 20480 c.s.p. $2\times10^3$-$2\times10^9$ conidia/ml

**7.** A formulation according to any of claims 4 to 6, for use with chemical insecticides, which have been found to be compatible, examples of which are Orytis, Dicarzol®, Lannate® and Canfidor® in a integrated pest management program.

**8.** A formulation according to any of claims 4 to 6, for use with Previcur® chemical fungicide at half the maximum concentration recommended by the manufacturer, in a integrated pest management program.

**Patentansprüche**

**1.** Fadenpilz *Beauveria bassiana* Stamm *Bbl,* hinterlegt unter CECT 20480, anpassungsfähig an einen basischen extremen pH-Wert von 10.

**2.** Fadenpilz nach Anspruch 1, anpassungsfähig an eine extreme Temperatur von 75°C während fünf Minuten.

**3.** Die Verwendung von *B. bassiana* Stamm *Bbl,* hinterlegt unter CECT 20480 als ein biologisches Schädlingsbekämpfungsmittel.

**4.** Formulierung, umfassend den Fadenpilz *B. bassiana* Stamm *Bbl,* hinterlegt unter CECT 20480, und einen Träger.

**5.** Flüssige Formulierung, deren quantitative Zusammensetzung wie folgt ist:

- Pflanzliche Öle 94-96 Gew.-%
- Inerte Komponenten 6-4 Gew.-%
- *Beauveria bassiana* CECT 20480 c.s.p. $2x10^3$-$2x10^9$ Konidien/ml

**6.** Feste Formulierung, zusammengesetzt aus einem Träger und einer Kombination von biologischen Komponenten, deren quantitative Zusammensetzung wie folgt ist:
*Träger*

- Protein 10-50 Gew.-%
- Fette 10-30 Gew.-%
- Zellulose 1-10 Gew.-%
- Laktose 10-60 Gew.-%
- Vitamin A 10000-30000 IE/kg
- Vitamin D-3 1000-10000 IE/kg
- Vitamin E 10-60 mg/Kg

- Kupfersulfat ($CuSO_4$ $5H_2O$) 5-20 mg/Kg

*Biologische Verbindungen*

- *Beauveria bassiana CECT 20480 c.s.p.* $2x10^3$-$2x10^9$ Konidien/ml

**7.** Formulierung nach einem der Ansprüche 4 bis 6 zur Verwendung mit chemischen Insektiziden, die sich als kompatibel erwiesen haben, z.B. Orytis®, Dicarzol®, Lannate® und Confidor®, in einem integrierten Schädlingsbekämpfungsprogramm.

**8.** Formulierung nach einem der Ansprüche 4 bis 6 zur Verwendung mit dem chemischen Fungizid Previcur® bei der Hälfte der maximalen Konzentration, die vom Hersteller empfohlen wird, in einem integrierten Schädlingsbekämpfungsprogramm.

**Revendications**

**1.** Champignon filamenteux Beauveria bassiana de la souche Bb1 déposé sous le numéro CECT 20480 adaptable à une valeur de pH extrême basique de 10.

**2.** Champignon filamenteux selon spécification 1 adaptable à une température extrême de 75°C pendant cinq minutes.

**3.** Utilisation de la souche Bb1 de B. Bassiana déposé sous le numéro CECT 20480 en qualité d'agent de contrôle biologique.

**4.** Formulation comprenant la souche Bb1 du champignon filamenteux B. Bassiana déposé sous le numéro CECT 20480 et un porteur.

**5.** Formulation liquide dont la composition quantitative est la suivante :

- Huiles végétales : 94 - 96 % en poids
- Composants inertes : 6 - 4 % en poids
- Beauveria bassiana CECT 20480 c.s.p. 2 x $10^3$ -2 x $10^9$ conidia / ml

**6.** Formulation solide composée d'un porteur et une formulation de composés biologiques dont la composition quantitative est la suivante:
Porteur :

- Protéine 10 - 50 % en poids
- Graisses 10- 30 % en poids
- Cellulose 1 - 10 % en poids
- Lactose 10 - 60 % en poids
- Vitamine A 10000 - 30000 UI /kg
- Vitamine D-3 1000 - 10000 UI / kg
- Vitamine E 10 - 60 mg / kg
- Sulfate de cuivre ($CUSO_4$ $5H_2O$) 5 - 20 mg / kg

Composants biologiques

- Beauveria bassiana CECT 20480 c.s.p. 2 x $10^3$ -2 x $10^9$ conidia /ml

**7.** Formulation selon toute spécification 4 à 6 pour un usage avec insecticides chimiques, qui se sont avérés compatibles, comme par exemple : Orytis®, Dicarzol®, Lannate® et Confidor® dans le cadre d'un programme de gestion des pesticides intégré.

**8.** Formulation selon n'importe quelle spécification de 4 à 6 pour un usage avec le fongicide chimique Previcur® à la moitié de la concentration maximum recommandée par le fabricant dans le cadre d'un programme de gestion des pesticides intégré.

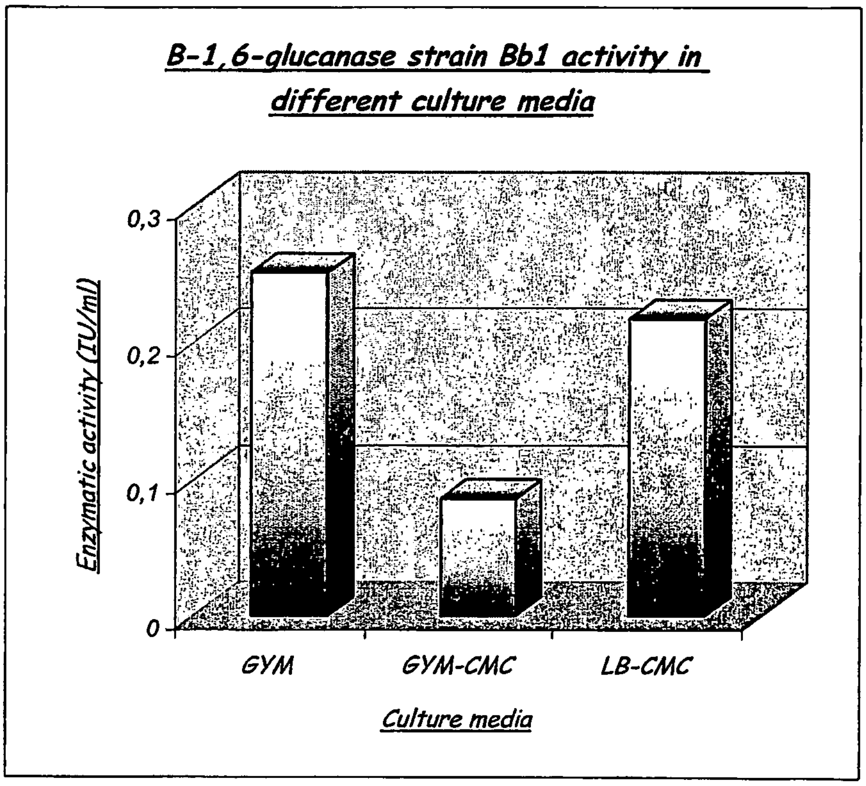
B-1,6-glucanase strain Bb1 activity in different culture media
0,3
0,2
0,1
0
Enzymatic activity (IU/ml)
GYM
GYM-CMC
LB-CMC
Culture media

Figure 1

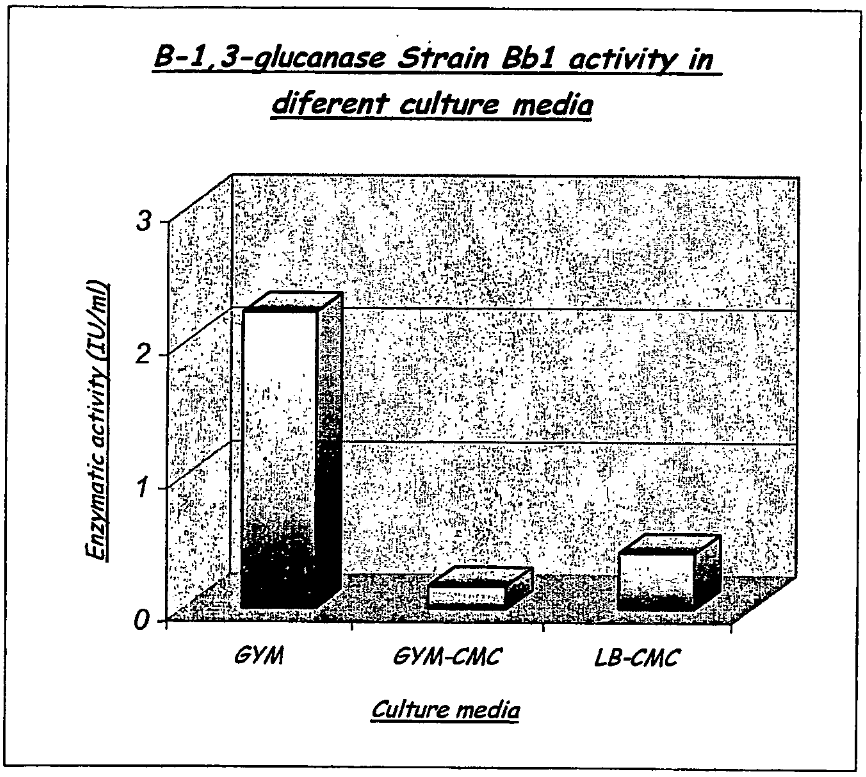
B-1,3-glucanase Strain Bb1 activity in diferent culture media
Enzymatic activity (IU/ml)
3
2
1
0
GYM
GYM-CMC
LB-CMC
Culture media

Figure 2

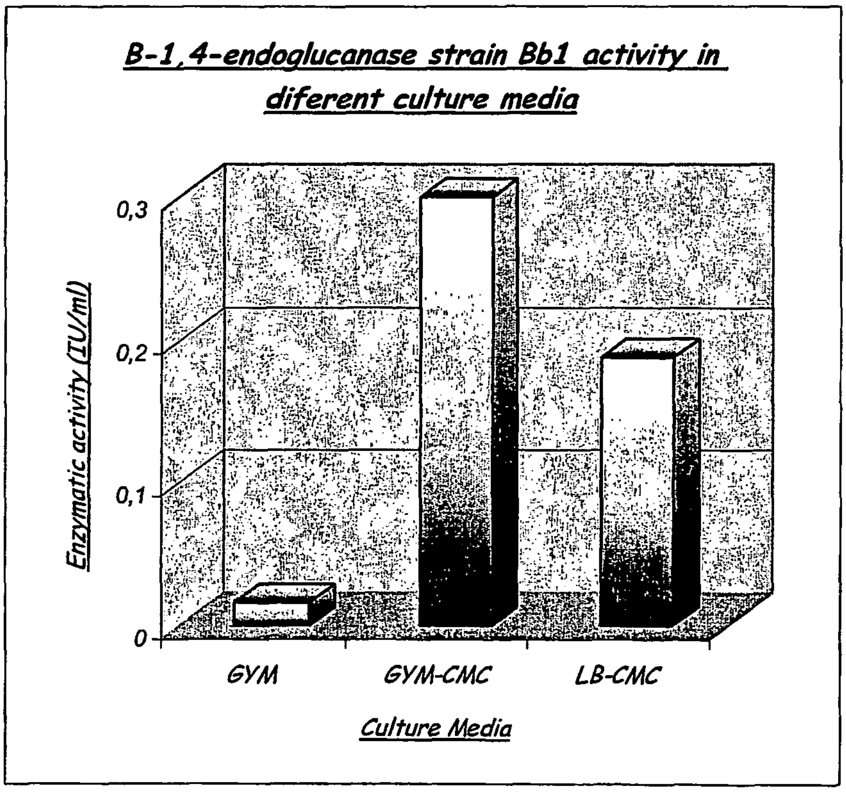
B-1,4-endoglucanase strain Bb1 activity in
diferent culture media
Enzymatic activity (IU/ml)
0,3
0,2
0,1
0
GYM
GYM-CMC
LB-CMC
Culture Media

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 0305440 W
- GB 0229127 A

### Non-patent literature cited in the description


- **FARIA MARCOS et al.** *Biological control of Bemisia tabaci with fungi*
- **KUHLS, K ; LIECKFELDT, E ; SAMUELS, G.J ; KOVACS, W ; MEYER, W ; PETRINI, O ; GAMS, W ; BÖRNER, T ; KUBICEK, C.P.** Molecular evidence that the asexual industrial fungus Trichoderma reesei is a clonal derivate of the ascomycete Hypocrea jecorina. *Proceedings of the National Academy of Sciences,* 1996
- **MULLIS, K.B ; FALLONA, F.A.** Specific synthesis of DNA in vitro via polymerase-catalyzed chain reaction. *Methods in Enzymology,* 1987, vol. 155, 335-350
- Colorimetric analysis of sugars. **NELSON, N.J.** Methods in enzymology. Academic Press, 1957, vol. III, 85-86
- **RAEDER , U ; BRODA, P.** Rapid preparation of DNA from filamentous fungi. *Letters Applied Microbiology,* 1985, vol. 1, 17-20
- **SAMBROOK, J ; FRITSCHE, E ; MANIATIS, T.** Molecular cloning: A laboratory manual. Cold Spring Harbor Laboratory, 1989
- **SOMOGYI, M.** Notes on sugar determination. *Journal Biological Chemistry,* 1952, vol. 195, 19-29
- Amplification and direct sequencing of fungal ribosomal RNA gens for phylogenetics. **WHITE, T.J ; BRUNS, T ; LEE, S ; TAYLOR, J et al.** PCR Protocols-A Guide to Methods and Applications. Academic Press, 1990, 315-322

SEQUENCE LISTING

<110> A.M.C. chemical S.L.
Akdi, Khalid
Pérez Barrera, Francisco
Exposito Cubero, carmelo
Gomis Garcia, Maria Dolores

<120> Biological control Agent And Formulations

<130> WPP287205

<140> PCT/GB03/05440
<141> 2003-12-15

<150> GB0229127.6
<151> 2002-12-13

<160> 5

<170> PatentIn version 3.1

<210> 1
<211> 18
<212> DNA
<213> Artificial sequence

<220>
<223> Primer
<400> 1
tcggtaggtg aacctgcg 18

<210> 2
<211> 20
<212> DNA
<213> Artificial sequence

<220>
<223> Primer
<400> 2
tcctccgctt attgatatgc 20

<210> 3
<211> 20

<212> DNA
<213> Artificial sequence

<220>
<223> Primer
<400> 3
gctgcgttct tcatcgatgc 20

<210> 4
<211> 20
<212> DNA
<213> Artificial sequence

<220>
<223> Primer
<400> 4
gcatcgatga agaacgcagc 20

<210> 5
<211> 605
<212> DNA
<213> Beauveria bassiana

<400> 5

```
cccttttcccc cgggcttttt taaatttggg aattaatggg cctttaaagt tttccagcgg    60
ggttagtcct aacctgaatt tcggaggtct aacgttcaag gaagttgggt gttttacggc    120
gtggccgcgt cggggttccg gtgcgagctg tattactacg cagaggtcgc cgcggacggg    180
ccgccactcc atttcagggc cggcggtgtg ctgccggtcc ccaacgccga cctcccccag    240
gggaggtcga gggttgaaat gacgctcgaa caggcatgcc cgccagaatg ctggcgggcg    300

caatgtgcgt tcaaagattc gatgattcac tggattctgc aattcacatt acttatcgca    360
tttcgctgcg ttcttcatcg atgccagagc caagagatcc gttgttgaaa gttttgattc    420
atttgttttg ccttgcggcg tattcagaag atgctggaat acaagagttt gaggtccccg    480
gcgggccgct ggtccagtcc gcgtccgggc tggggcgagt ccgccgaagc aacgataggt    540
aggttcacag aagggttagg gagttgaaaa ctcggtaatg atccctccgc aggttcacct    600
acgga                                                                605
```